(19) Europäisches Patentamt
European Patent Office
Office européen des brevets

(11) **EP 4 449 977 A1**

(12) **EUROPEAN PATENT APPLICATION**

(43) Date of publication:
**23.10.2024 Bulletin 2024/43**

(21) Application number: **23169081.9**

(22) Date of filing: **20.04.2023**

(51) International Patent Classification (IPC):
**A61B 5/00** *(2006.01)* **A61N 1/04** *(2006.01)*

(52) Cooperative Patent Classification (CPC):
**A61B 5/4076; A61N 1/0456; A61N 1/0472;
A61N 1/0484;** A61B 5/383; A61N 1/36025;
G16H 50/20; G16H 50/50

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **Université d'Aix Marseille**
**13007 Marseille (FR)**
• **Institut National de la Santé et de la Recherche
Médicale**
**75013 Paris (FR)**

(72) Inventors:
• **JIRSA, Viktor**
**13400 Marseille (FR)**
• **HASHEMI, Meysam**
**13005 MARSEILLE (FR)**
• **ARBABYAZD, Lucas**
**69100 Villeurbanne (FR)**
• **WILLIAMSON, Adam**
**13010 MARSEILLE (FR)**

(74) Representative: **Macquet, Christophe**
**Macquet & Associés
Arche des Dolines
7, rue Soutrane
06560 Sophia Antipolis (FR)**

(54) **METHOD FOR INFERRING A CHANGE IN A WHOLE-BRAIN NETWORK AND SYSTEM**

(57) The invention relates to a method for inferring a change in a whole-brain network in a brain of a mammal. The method comprises the steps of providing a computerized platform modelling various regions of a mammal brain and connectivity between these regions; providing three-dimensional anatomical structural imaging data of the brain of the mammal; providing a parametric mathematical neural model describing an evolution of the neural field in a brain of a mammal; providing magnetic resonance imaging time-series data of the brain; fitting the model with the magnetic resonance imaging time-series acquired; estimating the parameters of the model; inverting the model; and inferring the change in the whole-brain network in the mammal's brain.

FIG. 1

**Description**

FIELD OF THE INVENTION

The invention relates to a method for inferring a change in a whole-brain network in a brain of a mammal. It also relates to a system for the implementation of this method. The method according to the invention comprises, in particular, the steps of providing a computerized platform modelling various regions of a mammal brain and connectivity between these regions; providing three-dimensional anatomical structural imaging data of the brain of the mammal, the data comprising anatomical structural data relating to the structural connectivity in the brain of the mammal; and providing the computerized platform with the anatomical structural imaging data of the brain of the mammal.

BACKGROUND OF THE INVENTION

Structural connectivity (SC) refers to the white matter fiber tracts physically connecting grey matter brain regions. It can be measured non-invasively using Diffusion Weighted Imaging (DWI) techniques. During the aging process or due to pathologic conditions affecting the brain, e.g. Epilepsy, Alzheimer, Parkinson, tumor, stroke, traumatic injury, SC is reported to decrease or being perturbed, particularly with respect to the number of inter-hemispheric fibers. Given that SC is the underlying construct for brain regions to exchange information, it is generally hypothesized that SC changes modify functional activity dynamics and that the interplay between these two modalities may explain the changes in certain measured data.

So far, some data-driven methods based or statistical analysis have been applied to infer the causal relationship between the brain structure and function. Nevertheless, an efficient and accurate inference at whole-brain level is still lacking.

Employing mechanistic neural models accounting for the nonlinearity and the causality and the personalized brain, focusing on each subject anatomical information individually, may have appeared highly promising. However, merging whole-brain anatomical data with an exact mathematical model of neural populations imposes significant challenges to statistical inference. This is due to the large dimensionality of the search space and observations, nontrivial brain network effects, nonlinearity involved in spatiotemporal brain organization, and uncertainty in prior information. Moreover, the dynamics of source states are unobserved, in fact hidden, leading to non-identifiability issues for parameter estimation.

Virtual brain modeling of brain disease is a data-driven approach that combines personalized anatomical information with parametric mathematical models of abnormal brain activity to generate spatiotemporal patterns as observed in brain imaging signals. Virtual brain modeling has gained traction in applications of computational neuroscience, particularly in brain diseases. This is due to the interpretability of dynamical systems merged with personalized anatomical data, taking into consideration the laws of physics and biophysical properties of neural populations (mean-field modeling) while maintaining a level of tractability in the production of whole-brain imaging signals. Such a parametric formulation is equipped with a stochastic generative process, which itself provides the basis for inference and prediction on the local and global brain dynamics affected by disorders. However, the inverse problem of such a large-scale network model, with non-linear local brain dynamics, is challenging. In particular, the calculation of likelihood function becomes intractable. Thus, efficient sampling algorithms are required to estimate the model parameters pertaining to the hypothetical regions in the brain involved in the generation of disorders, ideally including the uncertainty for decision-making process.

Bayesian framework is a natural and principled way of combining prior information with observations to characterize uncertainty over unknown quantities. This probabilistic methodology provides an estimation of unknown model parameters by incorporating the uncertainty or variation in assumptions, latent variables, measurements, and algorithmic predictions. Markov Chain Monte Carlo (MCMC) methods comprise a class of algorithms for sampling from a probability distribution, which is a non-parametric process and asymptotically unbiased to sample from a complex posterior distribution. However, evaluation of the target distribution can be prohibitive in high-dimensional spaces, often with the rejection of many proposals that impose the search space exploration to converge very slowly. It is well-known that gradient-free sampling algorithms generally fail to explore the parameter space efficiently when applied to large-scale inverse problems, as often encountered in the application of whole-brain imaging for clinical diagnoses. In particular, the traditional MCMC mix poorly in high-dimensional parameter spaces involving correlated variables. In contrast, gradient-based algorithms, such as Hamiltonian Monte Carlo (HMC), although computationally expensive, are far superior to gradient-free sampling algorithms in terms of the number of independent samples produced per unit computational time. This class of sampling algorithms provides efficient convergence and exploration of parameter space even in very high-dimensional spaces that may exhibit strong correlations. Nevertheless, the efficiency of gradient-based sampling methods such as HMC is highly sensitive to the user-specified algorithm parameters. More advanced MCMC sampling algorithms such as No-U-Turn Sampler (NUTS), a self-tuning variant of HMC may solve these issues by adaptively tuning the algorithm parameters. Probabilistic Programming Languages (PPLs) may provide an efficient implementation for automatic Bayesian inference on user-defined probabilistic models by featuring the next generation of MCMC sampling and VI algorithms such as NUTS and ADVI, respectively. With the help of PPLs, these algorithms take the advantage of automatic differentiation methods for the computation of derivatives in computer programs to avoid the random walk behavior and sensitivity to correlated parameters. In particular, Stan™ is a high-level statistical modeling tool for Bayesian inference and probabilistic machine learning, which provides advanced inference algorithms such as NUTS and ADVI, enriched with extensive and reliable diagnostics. Although PPLs allow for automatic inference, the performance of these algorithms can be sensitive to the form of parameterization. An appropriate form of reparameterization in the probabilistic models of system dynamics, governed by a set of nonlinear stochastic differential equations, is a challenging problem for inference efficiency.

SUMMARY OF THE INVENTION

Accordingly, a need exists for inferring changes in a human or mammal whole brain structure-function relationship.

In accordance with a first aspect, the invention concerns a method for inferring a change in a whole-brain network in a mammal's brain, comprising

> providing a computerized platform modelling various regions of a mammal's brain and connectivity between these regions;
> providing three-dimensional anatomical structural imaging data of the mammal's brain, the data comprising anatomical structural data relating to a structural connectivity in the brain of the mammal;
> providing the computerized platform with the anatomical structural imaging data of the mammal's brain;
> providing a parametric mathematical neural model describing an evolution of a neural field in a brain of a mammal;
> providing magnetic resonance imaging time-series data of the mammal's brain, wherein said magnetic resonance imaging time-series data are acquired under stimulation of at least a specific region of the brain of the mammal;
> fitting the model with the magnetic resonance imaging time-series data;
> estimating the parameters of the model;
> inverting the model; and

inferring the change in the whole-brain network in the mammal's brain.

Advantageously, - the three-dimensional anatomical structural imaging data of the mammal's brain further comprise geometry data extracted from T1-weighted magnetic resonance imaging and wherein the structural connectivity is extracted from tractography on diffusion weighted magnetic resonance imaging data; - the magnetic resonance imaging time-series data of the mammal's brain are acquired non-invasively; - the magnetic resonance imaging time-series data of the mammal's brain are acquired non-invasively by performing a brain stimulation of the specific region of the brain of the mammal; - for performing the brain stimulation of the mammal's brain, it is provided at least two pairs of stimulation electrodes, each pair of electrodes providing an electric signal at a carrier frequency generating an envelope frequency for stimulation at a point in space in the brain of the patient, the envelope frequency being equal to the difference Δf between the two electric signal frequencies provided; - for performing the brain stimulation of the mammal's brain, it is provided at least four pairs of stimulation electrodes, each pair of electrodes providing an electric signal at a carrier frequency, wherein the mean value between the carrier frequency of a first pair of electrodes and the carrier frequency of any second pair of electrodes defines a first mean carrier frequency, and the mean value between the carrier frequency of a third pair of electrodes and the carrier frequency of a fourth pair of electrodes defines a second mean carrier frequency, and wherein the difference between said first and second mean carrier frequencies is at least 200 Hz, preferably at least 500 Hz even more preferably at least 800 Hz; - each electric signal provided by each pair of electrodes has a carrier frequency of at least 800 Hz, preferably at least 900 Hz, more preferably at least 1000 Hz; - the parameters of the model are estimated by performing an automatic full Bayesian inference; - the automatic full Bayesian inference is performed using Monte Carlo algorithms; - the automatic full Bayesian inference is performed using Probabilistic Programming languages; - the model depend on a stimulation to at least the specific brain region; the model is provided by the set of equations as follows:

$$\dot{r}_i(t) \quad = \quad \frac{\Delta}{\pi} + 2r_i(t)V_i(t)$$

$$\dot{v}_i(t) \;=\; V_i^2(t) - \pi^2 r_i^2(t) + \eta + J r_i(t) + G \sum_{j=1}^{N} SC_{ij}^*(r_j - r_i)) + I_{stim} + \xi(t),$$

where the variable $r(t)$ is the population firing rate of a region $i$ *of the brain,* and $v(t)$ is the average membrane potential of the mass in region i, the parameter $J$ represents the average synaptic weight, the parameters $\Delta$ and $\eta$ represent the average excitability of the mass neurons and the heterogeneous noise spread, respectively, and the parameter G is the global parameter that modulates the overall impact of $SC^*$ on the state dynamics of each region and represents the disorders in the brain connectome; - the parametric neural model describing the evolution of the neural field the brain of the mammal depends on at least a parameter that corresponds to the normalized intensity of changes in the structural connectivity and on a global scaling parameter; - the three-dimensional anatomical structural imaging data of the mammal's brain are parcelled into discrete regions; - the regions are non-overlapping; - the change is indicative of a brain disorder or of a condition affecting the mammal's brain, or of a severity of a brain disorder or of a condition affecting the mammal's brain.

In accordance with a second aspect, the invention concerns a method for diagnosing, assessing the severity or monitoring a brain disorder in a mammal's brain or a condition affecting the mammal's brain, comprising

providing a computerized platform modelling various regions of a mammal's brain and connectivity between these regions;
providing three-dimensional anatomical structural imaging data of the mammal's brain, the data comprising anatomical structural data relating to a structural connectivity in the brain of the mammal;
providing the computerized platform with the anatomical structural imaging data of the mammal's brain;
providing a parametric mathematical neural model describing an evolution of a neural field in a brain of a mammal;
providing magnetic resonance imaging time-series data of the mammal's brain, wherein said magnetic resonance imaging time-series data are acquired under stimulation of at least a specific region of the brain of the mammal;
fitting the model with the magnetic resonance imaging time-series data;
estimating the parameters of the model;
inverting the model; and

diagnosing, assessing the severity or monitoring a brain disorder in the mammal's brain or a condition affecting the mammal's brain.

Preferentially, the brain disorder or the condition affecting the mammal's brain is a pathologic condition affecting the brain selected from the group consisting in epilepsy, Alzheimer, Parkinson, multiple sclerosis, brain tumor including glioblastoma, and a condition resulting from a stroke or a traumatic injury.

In accordance with a third aspect, the invention concerns a system for inferring changes in a whole-brain network in a brain of a mammal, comprising

a computerized platform modelling various regions of a mammal's brain and connectivity between these regions;
three-dimensional anatomical structural imaging data of the brain of the mammal, the data comprising anatomical structural data relating to a structural connectivity in the brain of the mammal;
the computerized platform being provided with the anatomical structural imaging data of the brain of the mammal;
a parametric mathematical neural model describing an evolution of the neural field in a brain of a mammal;
magnetic resonance imaging time-series data of the brain of the mammal, wherein said magnetic resonance imaging time-series data are acquired under stimulation of at least a specific region of the brain of the mammal;
the model being fitted with the magnetic resonance imaging time-series data;
means for estimating the parameters of the model;
means for inverting the model; and

means for inferring changes in the whole-brain whole-brain network in the mammal's brain.

BRIEF DESCRIPTION OF THE DRAWINGS

Other features and aspects of the present invention will be apparent from the following description and the accompanying drawings, in which

Fig. 1 illustrates various steps of the method according to the invention;
Fig. 2A illustrates the principle of a brain stimulation for the implementation of the method according to the invention;

Figs. 2B and 2C illustrate an example of a mode for carrying out a brain stimulation for the implementation of the method according to the invention, in which 4 pairs of electrodes are used, defining a quadrupole, those pairs of electrodes being noted E1A/E1B, E2A/E2B, E3A/E3B and E4A/E4B in Fig. 2C;

Fig. 2D compares the modulation the index profiles of dipole and octupole configurations for brain stimulation, for the implementation of the invention;

Figs. 3A to 3D illustrate the personalized structural connectivity matrix that may be used for the virtual modeling of disorders using a mask-based approach, as the anatomical driver of a brain disease, according to the invention;

Figs.4A to 4E illustrate the Bayesian inference on SC changes using the mask-based approach with HMC according to the invention. In Figs. 4A and 4B, it is shown, in dark gray, the simulated and, in light gray, the fitted BOLD time-series given virtual disordered SC, without and with the stimulation on brain regions, respectively.

## DETAILED DESCRIPTION OF THE INVENTION

The invention proposes a method which, in particular, addresses the inference issues at whole-brain level with automatic Monte Carlo sampling algorithms that allow inferring the changes in brain structure-function relationship under stimulation in certain regions according to clinical evidence.

The method according to the invention is a method for inferring a change, for example disordered change, in a whole-brain network in a mammal's brain, in particular a human brain.

The whole-brain network includes connectivity and, preferentially, structural connectivity and/or network nodes of a whole-brain. The structural connectivity refers in particular to the white matter fiber tracts physically connecting grey matter brain regions. The changes are related to a pathological condition that comprises a deterioration of the connectivity or of various regions of the brain. The changes may include changes in the whole-brain network of the mammal's brain at a time in comparison with the whole-brain network of the mammal's brain inferred at a previous time or a deviation in the whole-brain network of the mammal's brain in comparison with a whole-brain network of a mammal's reference brain. The changes are indicative of a brain disorder or of a condition affecting the mammal's brain, or of a severity of a brain disorder or of a condition affecting the mammal's brain. The brain disorder or the condition affecting the mammal's brain is a pathologic condition affecting the brain selected from the group consisting in epilepsy, Alzheimer, Parkinson, multiple sclerosis, brain tumor including glioblastoma, and a condition resulting from a stroke or a traumatic injury.

This method comprises a step of providing a computerized platform modelling various regions of a mammal brain and connectivity between these regions. Three-dimensional anatomical structural imaging data of the brain of the mammal are also provided, these data comprising anatomical structural data relating to the structural connectivity in the brain of the mammal. The computerized platform is personalized according to the anatomical structural imaging data of the brain of the mammal. Further, a parametric mathematical neural model describing an evolution of the neural field in a brain of a mammal is provided. Also, first magnetic resonance imaging time-series data of the brain of the mammal and second magnetic resonance imaging time-series data of the brain of the mammal are provided, the second magnetic resonance imaging time-series data being provided under stimulation of at least a specific region or area of the brain of the mammal. The model is then fitted with the magnetic resonance imaging time-series acquired. The parameters of the model are estimated and the model is inverted. That allows to infer the disordered changes in the whole-brain structural connectivity in the brain of the mammal.

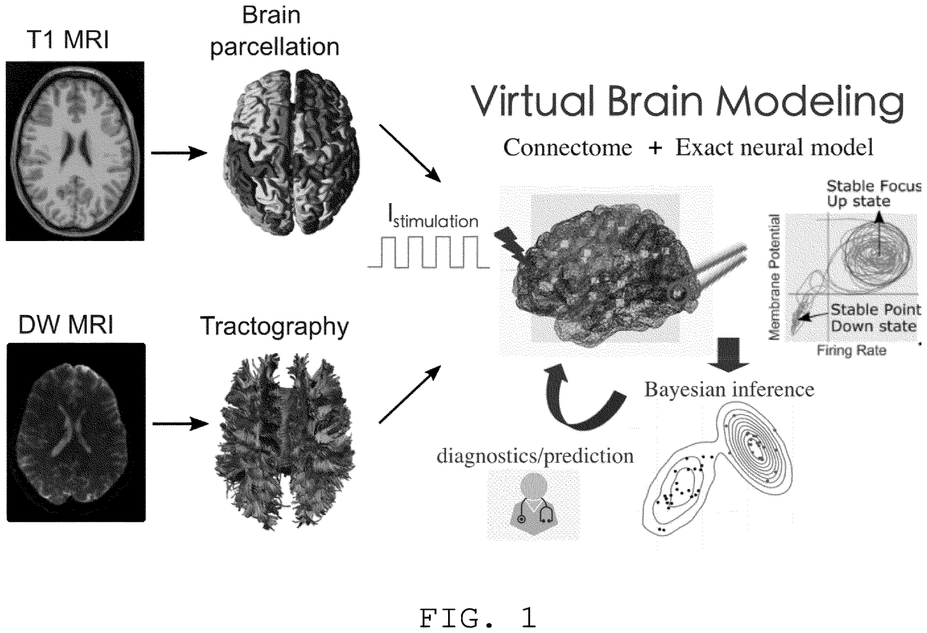

Some of the above steps according to the method of the invention are illustrated in Fig. 1. As appearing in Fig. 1, the three-dimensional anatomical structural imaging data of the brain of the mammal comprise geometry data, that is extracted from T1-weighted magnetic resonance imaging. The structural connectivity is extracted from tractography on diffusion weighted magnetic resonance imaging data.

The invention allows to estimate the posterior distribution of parameters in Virtual Brain Models given the stimulation to certain brain regions for increasing the causal evidence in the structure-function relationship, due to the change in brain organization relying on a mask-based approach. The invention integrates the non-invasive patient specific data, a mask-based approach, and automatic model inversion to estimate the posterior distribution of parameters in virtual brain models given the stimulation to certain brain regions for increasing the causal evidence in the structure-function relationship, due to the change in brain organization. The full Bayesian inference is performed using self-tuning Monte Carlo algorithms, monitored with convergence diagnostics, and the code is implemented in Stan™ language for statistical modeling and automatic machine learning.

Sampling algorithms embedded in PPLs are then used according to the invention to automatically perform fully Bayesian inference and efficiently retrieve the statistical relationships between parameters and observations. More importantly, efficient parameterization is performed for HMC sampling while brain stimulation to remove the parameter degeneracy. The invention is able to accurately and efficiently estimate the posterior distribution of parameters linked to brain pathologic conditions. The presented Bayesian methodology according to the invention can deal with non-linear latent dynamics and parameter degeneracy, paving the way for reliable prediction on brain disorders from neuroimaging modalities which can be crucial for planning intervention strategies.

According to the invention, the brain stimulation is introduced, in particular, to solve the issue of degeneracy, i.e. the non-identifiability, allowing to accurately predict the perturbation in patient-specific connectome caused by brain pathologic conditions. Taking advantage of stimulation in virtual brain model and the recent advances in probabilistic programming languages for automatic Bayesian inference, the invention provides the feasibility and efficiency to estimate the model parameters pertaining to the hypothetical regions and brain reorganization involved in the generation of brain disorders, including the uncertainty for decision-making process.

The stimulation of the mammal's brain is non-therapeutic. It is an external stimulation, that is targeted to a specific region of the brain, which may be located deeply in the brain. The stimulation could be implemented by using implantable stimulation electrodes such as deep brain stimulation (DBS) electrodes that are MRI-compatible, but is preferentially implemented non-invasively using non-implanted electrodes as in multi-modal temporal interference stimulation, which is described hereafter, and allows to stimulate specific brain regions, in a focalized and controlled manner.

**Non-invasive Brain Stimulation - Temporal Interference (TI) and multi-modal Temporal Interference (mTI)**

The magnetic resonance imaging time-series data of the brain of the mammal are acquired non-invasively by performing a brain stimulation implementing temporal interference (TI) of specific regions of the brain of the mammal.

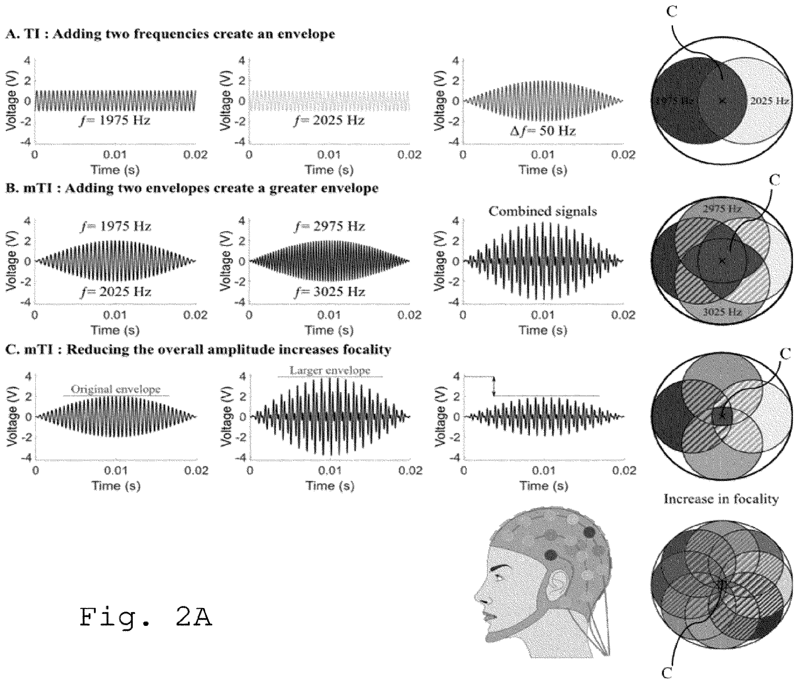

The brain stimulation technique implementing TI is non-invasive. It relies on the ability of high frequency currents to penetrate living tissue relatively unhindered compared to low frequency currents, inferior to 800 Hz, as tissue permittivity tend to increase with frequency. The TI principle is based on the interaction of two high frequency electric currents - or signals - each of them being provided at a carrier frequency of at least 900 Hz, preferably of at least 1000 Hz, but suffering from a slight difference. This frequency difference creates a temporal interference in a zone C that is both predictable and regular, at a frequency equal to the difference between two carrier frequencies, as seen in Fig. 2A, panel A wherein the carrier frequencies are of 1975 Hz and 2025 Hz and the frequency difference is of 50 Hz. The neuronal or muscular tissues will only be modulated when they are simultaneously stimulated by these two frequencies, namely when the tissue experiences the interference, as it is generally accepted that frequencies above 900 Hz should not stimulate tissue.

It is to be noted that all the tissue, which experiences the interference, can be modulated when the amplitude of the envelope exceeds a threshold. However, using only two pairs of electrodes offers little freedom in the spatial configuration of the stimulators, which often results in a large area of effective stimulation, especially in deep structures. In order to avoid to modulate unwanted nodes in the brains or neighboring nerves in the case of peripheral nerve stimulation, the number of stimulating pairs is preferentially increased to improve the focality of stimulation, namely the ability to modulate a targeted area without disturbing the surrounding area. This relies on the addition of several envelopes having the maximum amplitude of envelope at the same spot, as shown in Fig. 2A, Panel B.

With supplementary pairs of stimulation electrodes, the current provided by each pair of electrodes can be turned down to achieve the same effect at the targeted zone C, while offering a better focality, as shown in Fig. 2A, Panel C. When performing this multipolar interference technique, noted mTI in Fig. 2A, a few things should however be considered. The frequency sets chosen to create envelopes should respect certain criteria: each envelope should be of the same frequency, the difference between the means of the carrier frequencies of associated pairs used to create the envelopes should be at least 200 Hz, in order to prevent the creation of unwanted interferences. The phase of each envelope should be considered at the targeted zone. It should be synchronous at this targeted zone to deliver the most efficient stimulation, but asynchronous outside the targeted zone, which will decrease the efficiency of stimulation, thus also improving the focality of stimulation. This technique works better when the stimulating pairs are in different planes, allowing to really "lock" the targeted zone in 3 dimensions. mTI allows the brain stimulation to be focal and to reach deep brain areas.

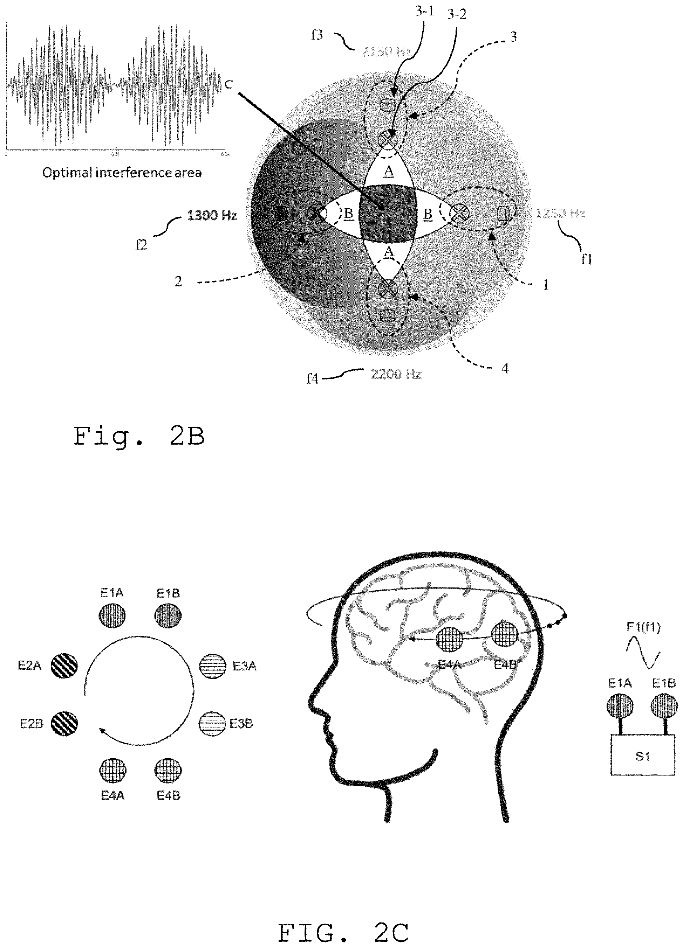

Fig. 2B illustrates an embodiment of a brain stimulation system for acquiring functional data recordings representative of the brain, and implementing mTI. As illustrated in Fig. 2B, the system comprises 4 stimulation pairs of electrodes 1, 2, 3, 4, a first stimulation pair of electrodes 1, a second stimulation pair of electrodes 2, a third stimulation pair of electrodes 3, and a fourth stimulation pair of electrodes 4, defining a quadrupole. The pairs of electrodes are positioned on different sides of the brain scalp, each stimulation pair comprising one electrode 3-1 and its associated return electrode 3-2. It is to be noted that, in Fig. 2B, the electrodes of a pair of electrodes are schematized by a cylinder and a circle containing a cross. For example, the stimulation pair of electrodes referenced 3 in Fig. 2B comprises two electrodes that are referenced 3-1 and 3-2. The electrode 3-2, that is schematized by a circle containing a cross, is the return electrode.

Each pair of electrodes provides an electric signal at a carrier frequency. The carrier frequency of the first stimulating pair of electrodes 1 is of 1250 Hz. The carrier frequency of the second stimulation pair 2 of electrodes is 1300 Hz. The carrier frequency of the third stimulation pair of electrodes 3 is 2150 Hz. The carrier frequency of the fourth stimulation pair of electrodes 4 is 2200 Hz. Hence, the carrier frequencies of the first 1 and second 2 stimulation pairs of electrodes are close one to another, with a difference in frequency of 50 Hz and the carrier frequencies of the third 3 and fourth 4 stimulation pairs of electrodes are close one to another, also with a difference in frequency of 50 Hz. The first 1 and second 2 stimulation pairs of electrodes are thus associated to generate a stimulation in a zone A of the brain that is located in between the pairs 1, 2 of electrodes. In other words, the electric signals provided by the first 1 and the second 2 pairs of electrodes generate a first envelope frequency in a zone A that is equal to the difference between the carrier frequencies of the signals provided by the first and second pair of electrodes 1, 2, i.e. 50 Hz. In the same way, the third 3 and fourth 4 stimulation pairs of electrodes are associated to generate a stimulation in a zone B of the brain that is located in between the stimulation pairs of electrodes 3 and 4. In other words, the electric signals provided by the third 3 and the fourth 4 pair of electrodes generate a second envelope frequency in a zone B that is equal to the difference between the carrier frequencies of the signals provided by the third and fourth pair of electrodes (3,4), i.e. 50 Hz.

The central zone C, that intersects the zones A and B, correspond to the optimal interference area, which is aimed to be stimulated. The stimulation frequency delivered to the optimal interference area C is equal to 50 Hz.

In Fig. 2B, the mean carrier frequency of the first 1 and second 2 stimulation pairs of electrodes is of 1275 Hz. The mean frequency of the third 3 and fourth 4 stimulation pairs of the electrodes is of 2175 Hz. The difference of the mean frequencies of, on one hand, the third and fourth stimulation pairs of electrodes and, on the other hand, the first and the second stimulation pairs of electrodes, is of 2175 - 1275 = 900 Hz. This difference avoids the generation of unwanted stimulation areas at the periphery of the central zone C. A single focal zone is created inside the brain for the stimulation.

On a general point of view, it will be noted that:

advantageously, the carrier frequency of a stimulation pair of electrodes is of at least 800 Hz, preferentially of at least 900 Hz, and more preferentially of at least 1000 Hz;
advantageously, the difference between the carrier frequencies of two stimulating pairs of electrodes that are associated is of at least 25 Hz, but equal or less than 200 Hz. It is for example equal to exactly 50 Hz in Fig. 1B; and
advantageously, in order to avoid unwanted stimulation areas, the difference of the mean frequencies of, on one hand, a first couple of stimulation pairs of electrodes and, on the other hand, a second couple of stimulation pairs of electrodes, is of at least 200 Hz, preferably of at least 500 Hz and even more preferably of at least 800 Hz, for example 900 Hz.

A system according to the invention is also illustrated in Fig. 2C. As appearing on that figure, the system implements stimulation electrodes E in pairs, which are referenced E1A/E1B, E2A/E2B, E3A/E3B and E4A/E4B. There are 4 stimulation electrode pairs defining a quadrupole. These stimulation electrode pairs are positioned on the surface of the scalp, around the head of a mammal, for instance a human individual. The position of the electrodes of a pair of electrodes may be close one to another, even at an immediate vicinity, or at various positions, for instance, at opposite positions around the patient's head. However, the positioning of the electrodes should advantageously be calculated beforehand in such a way as to have a maximum modulation at the point of interest within the brain, while minimizing the electric current delivered by each electrode. If the situation allows it, positioning electrodes on various axes, such as the axes anterior-posterior, medio-lateral or dorsa-ventral, should result in a greater focal gain.

It is also to be noted that the electrodes should not act as capacitors during stimulation in order to avoid non-faradaic processes. They should be MRI-compatible. Typical Electrocardiogram (ECG) electrodes, which are composed of Ag/AgCl or electrodes coated with Poly(3,4-ethylenedioxythiophene) (PEDOT)-Poly(styrene sulfonate) (PSS) may be used according to the invention.

Any signal generator may be implemented in the system according to the invention, as long as it can generate high frequencies as disclosed herein, and it is possible to modify the phase of the signal. Generally, the generator is connected to one or more electric current sources. The current sources themselves electrically connected to the electrodes. Each pair of electrodes is connected to an individual signal source and the highest stimulation intensity is then concentrated between the electrodes of each such pair. As shown in Fig. 2C, each pair of electrodes is connected to a current source S1 for the first pair of electrodes E1A/E1B, S2 for the second pair of electrodes E2A/E2B, S3 for the third pair of electrodes and S4 for the fourth pair of electrodes. Only the current source S1 is illustrated in Fig. 1C. The current sources are independent. The current source S1 provides a signal function F1 from pair E1. The current source S2 provides a signal source F2 from pair E2. The current source S3 provides a signal function F2 from pair E3 and the current source S4 provide a signal function from pair E4. The signal functions are with corresponding frequencies f1, f2, f3 and f4. The signal function F can be any periodic signal such as sine wave, square wave, monopolar or bipolar pulses, with the frequency f (f1 for F1, f2 for F2, f3 for F3 and f4 for F4.

Focality gain using mTI scales to human brains, but also to smaller animal brains.

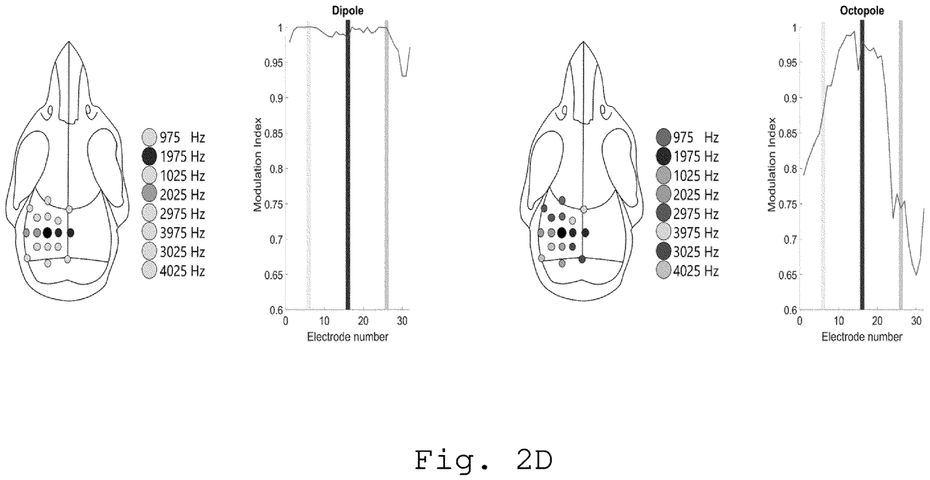

Indeed, as shown in Fig. 2D, standard TI (using only 2 pairs of electrodes) was performed in the mouse brain and compared to mTI, implemented by an octupole, i.e. 8 pairs of electrodes. The envelope amplitude is depicted using the modulation index.

As shown in the left part of Fig. 2D, the two pairs of electrodes provided currents at two frequencies and the envelope amplitude depicted using the modulation index. It is not possible to implant numerous SEEG electrodes in the mouse, therefore a silicon depth probe was implanted through the hippocampal target. The 32 electrodes do not show important variations in modulation index for this electrode configuration, indicating that, for the given exposure configuration, the stimulation reaches the hippocampal target but is not particularly focal. As further seen in Fig. 2D, mTI (8 pairs of electrodes providing 8 frequencies generating 4 overlapping envelopes) increases the focality compared to standard TI. Similarly, the focality significantly increases, as evidenced by the reduction of modulation index outside of the hippocampal target (around electrode 16 and below).

On a general point of view, the brain stimulation system comprises at least two, but advantageously at least four stimulation pairs of electrodes, each stimulation pair of electrodes providing an electric signal at a carrier frequency, the mean value between the carrier frequency of a first stimulation pair of electrodes and the carrier frequency of a second stimulation pair of electrodes defining a first mean carrier frequency, the mean value between the carrier frequency of a third stimulation pair of electrodes and the carrier frequency of a fourth stimulation pair of electrodes defining a second mean carrier frequency, and wherein the difference between said first and second mean carrier frequencies is equal to or greater than 200 Hz.

Preferentially:

- the first pair of electrodes (1) and the second pair or electrodes (2) generate a first envelope frequency (A), said first envelope frequency being equal to the difference between the electric signal (f1) frequency provided by the first pair of electrodes (1) and the electric signal frequency (f2) provided by the second pair of electrodes (2); the third pair of electrodes (3) and the fourth pair of electrodes (4) generate a second envelope frequency (B), said second envelope frequency being equal to the difference between the electric signal frequency (f1) provided by the third pair of electrodes (3) and the electric signal (f2) frequency provided by the fourth pair of electrodes (4); and wherein, the first envelope frequency is equal to the second envelope frequency.
- the system comprising between two and a number n of stimulation pairs of electrodes, preferably between four and a number n of stimulation pairs of electrodes, even more preferably height pairs of electrode; the difference between the carrier frequency of a first stimulation pair of electrodes and the carrier frequency of a second stimulation pair of electrodes defining a first envelope frequency; the difference between the carrier frequency of a third stimulation pair of electrodes and the carrier frequency of a fourth stimulation pair of electrodes defining a second envelope frequency; the difference between the carrier frequency of a subsequent stimulation pair of electrodes and the carrier frequency of another subsequent stimulation pair of electrodes defining a subsequent nth envelope frequency, and so on, the values of the first, second, and any subsequent nth envelope frequencies are equal, being especially less than or equal to 500 Hz, being especially equal to between 180 Hz and 1 Hz.
- the carrier frequencies of the stimulation pairs of electrodes are equal or greater than 900 Hz.
- the difference between any two mean carrier frequencies is equal to or greater than 800 Hz, preferably 900 Hz.
- for example, the carrier frequency of a first stimulation pair of electrodes is 1250 Hz, the carrier frequency of a second stimulation pair of electrodes is 1300 Hz, the carrier frequency of a third stimulation pair of electrodes is 2150 Hz, and the carrier frequency of a fourth stimulation pair of electrodes is 2200 Hz.
- the system is configured so that the phase of the envelopes is controlled, especially by using a predefined phase modulation.
- the stimulation amplitude of the electrodes is comprised between 10 $\mu$A and 2500 $\mu$A, better between 1 mA and 2 mA.
- a current is applied to the stimulation pairs of electrodes.
- the system further comprises one or more current sources, the one or more current sources being electrically connected to the pairs of electrodes.

**Material and Methods**

Based on a set of exact macroscopic equations for a network of spiking neurons (Montbrió, Ernest, Diego Pazó, and Alex Roxin. "Macroscopic description for networks of spiking neurons." Physical Review X 5.2 (2015): 021028) a virtual brain model was built by placing this class of neural mass models at parcelled brain regions, while each region is connected to other regions via the patient-specific SC matrix. Such a whole-brain modeling approach combines the mean-field model of neuronal activity with the subject-specific brain's anatomical information derived from non-invasive diffusion neuroimaging techniques (MRI, DTI). This allows to simulate various spatiotemporal patterns of brain activity for a specific patient in a single computational framework. Using The Virtual Brain (TVB) open-access platform, functional dynamics were simulated for a specific patient to reveal the impact of the SC changes on brain dynamics.

Synthetic data can provide the ground-truth of changes in SC, which is used to validate the inference results. The estimation was validated using Hamiltonian Monte Carlo (HMC) sampling. Notably, Bayesian inference provides confidence intervals that characterize the individual's uncertainty to be modeled by TVB allowing to detect the parameter degeneracy.

### Virtual pathological changes in brain connectome

The whole-brain was parceled into 400 discrete and nonoverlapping regions using a predefined 17-network parcellation scheme. The components provided by the 17-network parcellation can be allocated to the 7-network scheme, the latter being associated with seven distinct behavioral systems: visual, sensorimotor, limbic, frontoparietal, default-mode, dorsal and ventral-attention network.

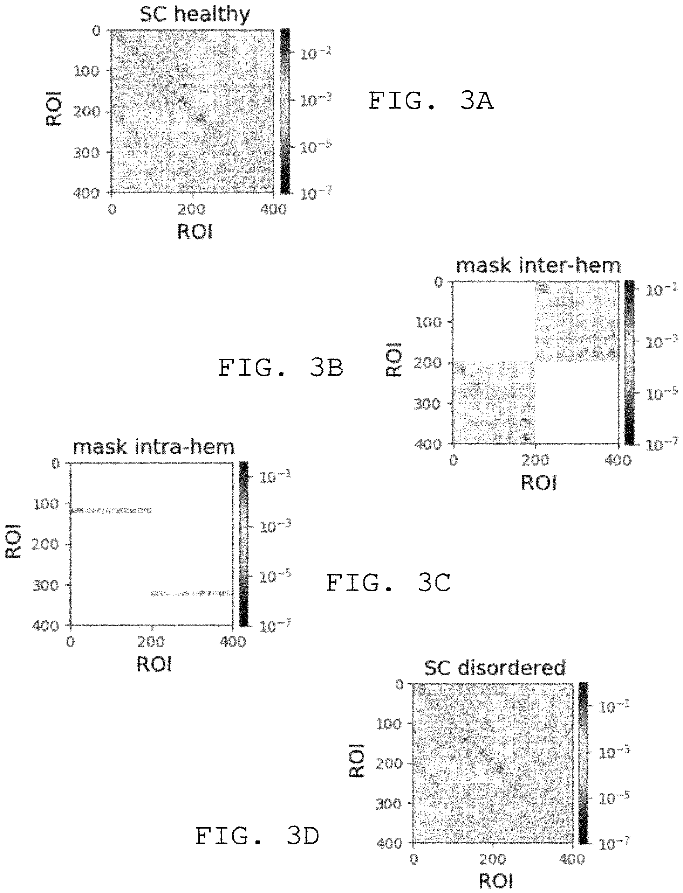

The approach to virtually model the disordered SC provides the basis to investigate whether a specific modification in SC affects the observed brain function in a causal sense. Therefore, as a preliminary investigation, the empirical SC datasets were inspected with regards to and specifically tested whether the previously reported strong disease related inter-hemispheric and intra-hemispheric SC (Figs. 3A to 3D). In particular, two masks were applied on the empirical connectome alpha and beta in the Equation No. 1 as follows:

$$SC_{disordered} = SC_{healthy} - \alpha * Mask_{inter} - \beta * Mask_{intra}$$

where parameters *alpha* and *beta* are the unknown normalized intensity of changes in SC (as the target of parameter estimation) and * represents an element-wise product (see Fig. 3A to 3D). The parameters alpha and beta spanned in the range [0-1]. In Bayesian setting, a weakly informative was placed as N(0.5, 0.5) on these parameters, given ground-truth 0.5 used for simulation.

Figs. 3A to 3D illustrate the personalized structural connectivity (SC) matrix used for virtual modeling of disorders using the mask-based approach, as the anatomical driver of the brain disease. In Fig. 3A, it is shown the SC matrix of a healthy subject. In Fig. 3B, a mask is provided on the inter-hemispheric regions. In Fig. 3C, a mask is provided on the intra-hemispheric regions. In Fig. 3D, the disordered SC given the inter- and intra-hemispheric masks, as in Equation No. 1.

### The virtual brain network model

To ensure a realistic in-silico experiment and therefore assess the function-structure link during the brain disorders via whole-brain modeling, resting-state activity was simulated via a brain network model that couples Neural Mass (NM) models described in (above-cited Montbrió et al., 2015) through the weighted edges in the SC matrix. The nodal dynamics is an exact mean-field representation of an ensemble of infinite all-to-all connected Quadratic-Integrate-and-Fire (QIF) neurons at the thermodynamic limit. Assuming that input currents to the mass system were distributed according to a Lorentzian distribution, the dynamics of each region was given by the following Equations No. 2:

$$\dot{r_i}(t) \quad = \quad \frac{\Delta}{\pi} + 2r_i(t)V_i(t)$$

$$\dot{v_i}(t) \quad = \quad V_i^2(t) - \pi^2 r_i^2(t) + \eta + Jr_i(t) + G \sum_{j=1}^{N} SC_{ij}^*(r_j - r_i)) + I_{stim} + \xi(t),$$

where the variable *r(t)* is the population firing rate of region *i*, and *v(t)* is the average membrane potential of the mass in region *i*. The parameter *J* represents the average synaptic weight, while the parameter *Delta* and *Eta* represent the average excitability of the mass neurons and the heterogeneous noise spread, respectively. These two parameters represent the center and the half-width of the Lorentzian distribution of the input currents. The values of these three parameters were set to J=14.5, Eta= -4.6, Delta= 0.7, in order to obtain a bi-stable regime, meaning a down-state fixed point and an up-state stable focus in the phase space. The bi-stability is a fundamental property to ensure a switching behavior in the data, that is considered representative of realistic dynamics of real data. The dynamics of a node can arise by the oscillation between these two points (the down-state and the up-state) in the phase space thanks to input current. The parameter G is the global parameter that modulates the overall impact of *SC*$^*$ on the state dynamics of each region and represents the disorders in the brain connectome given by Equation No. 1. The noise variable ensures the oscillations between the up-state and down-state and is distributed according to a gaussian distribution with a variance set to 0.03. The parameter *I_stim* represents the stimulation (step current) to selected brain regions.

The direct numerical integration for Equations No. 2 was performed via Heun-stochastic integration implemented in TVB open-source code. For each region, the equations were integrated with a variable time step (from 0.005 ms to 0.0005 ms) for 5 minutes and the variables r and v were down-sampled to the sampling frequency fs = 100 Hz. The time step was variable to adapt the integration process and avoid numerical errors or instabilities and the neural mass field was filtered via Balloon-Windkessel model to emulate the fMRI time series with repetition time TR set to 2000 ms.

**Model fitting**

To assess the effect of inter- and intra-hemispheric SC degeneration on the brain dynamics in a causal sense, the brain network model was applied in two scenarios. First, the simulated time series were fitted with disordered SC but no brain stimulation, then, subject to stimulation within certain regions of the brain (e.g., frontal regions or limbic system). In each case, three parameters *alpha, beta,* and global scaling parameter G were the target of estimation.

The model fitting was performed using STAN languages, and a self-tuning variant of HMC known as NUTS, with 200 warm-up, 200 sampling and acceptance rate of 0.95. Importantly, the convergence diagnostics such as \hat R metric and posterior behavior analysis validate the reliability of the estimations. The \hat R diagnostic provides an estimate of how much variance could be reduced by running chains longer. Each MCMC estimation has statistic associated with it, which is essentially the ratio of between-chain variance to within-chain variance. If is approximately less than 1.1, the MCMC convergence has been achieved (approaching to 1.0 in the case of infinite samples); otherwise, the chains need to be run longer.

From Bayesian perspective, if the posterior distribution follows the prior with no shrinkages with no statistical relation between the posterior samples of such parameters, they are non- structurally identifiable (in a simple example, inference over an extraneous parameter b that is not included in the model *c = a*). In contrast, if there is shrinkage in posterior but with a statistical relationship (manifold) in parameter space between the sampled parameters, they are practically non-identifiable (e.g., inference over parameters a and b from *c = a + b* or *c = ab,* manifesting as a high correlation in joint posterior samples).

**Results**

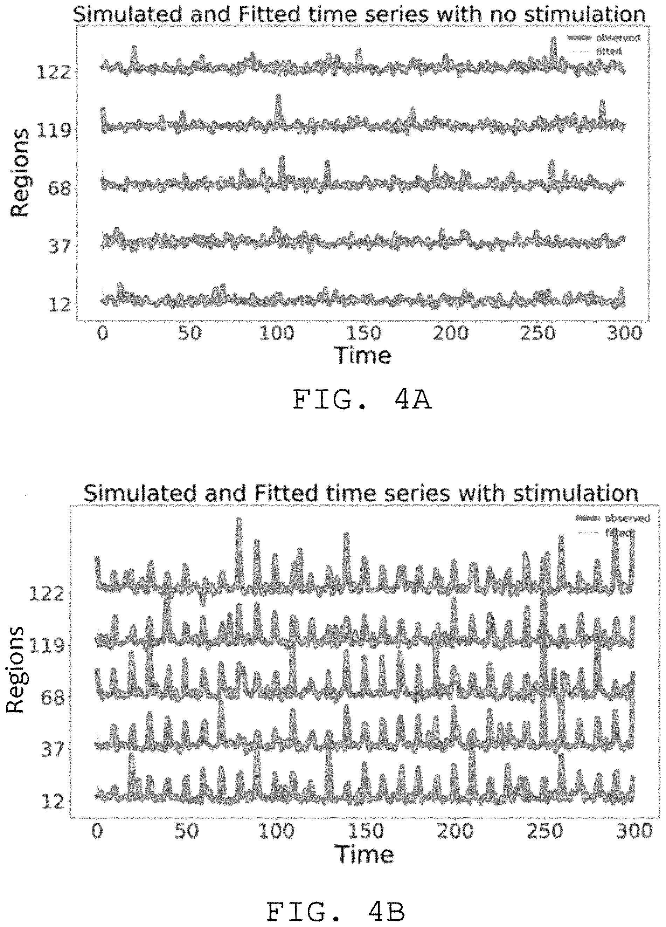

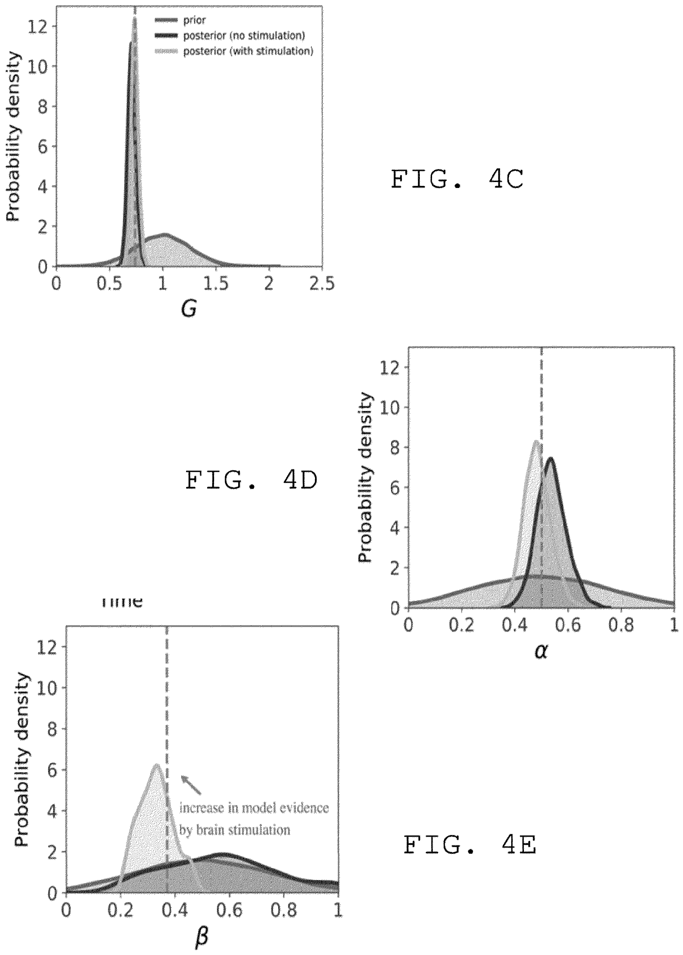

Synthetic data can provide us with the ground-truth of changes in SC, which can be used to validate the inference results. To this end, BOLD signals were generated as observations from the whole-brain modeling approach (Eq. 2) to perform inference on SC changes. Here it was considered the virtual disordered SC of a random subject, given by Eq 1., shown in Fig 3D. As illustrated in Fig. 4A, the fitted time series using HMC are in very good agreement with the observation. The estimated posterior for parameters G (global scaling parameter), alpha (inter-hemispheric mask), and beta (intra-hemispheric mask) are shown in Figs. 4C-4E, respectively (in red). It can be seen that the HMC provides a high level of certainty in the estimation of parameters G and alpha, but the posterior distribution of beta follows the prior with no shrinkages. This indicates non-identifiability in the estimation of intra-hemispheric mask. Here non-informative priors were used for Bayesian estimation. Although placing informative priors would mask the issue of non-identifiability, such information can be derived from complementary data such in MRI or clinical evidence that suggest restrictions on the possible range of a particular parameter, or a relationship between parameters. Moreover, adding information through prior can dramatically improve the exploration of search space in terms of computational cost and algorithmic diagnostics such as effective numbers of samples rendering the inference more efficient. Nevertheless, rather than placing informative prior to bias the estimation, information was added in the observation through stimulation of intra-hemispheric (here frontal) regions. The observed and fitted time series given the brain stimulation on selected regions are demonstrated in Fig 4B. The estimated posteriors for this case are shown in Fig. 4C-4E. It can be observed that brain stimulation provides a reasonable amount of uncertainty for estimation of virtual model parameters, in particular parameter beta (intra-hemispheric mask), thus it becomes identifiable.

***Conclusion***

Taking advantage of stimulation in virtual brain model and the recent advances in probabilistic programming languages for automatic Bayesian inference, our novel approach provides the feasibility and efficiency to estimate the model parameters pertaining to the hypothetical regions and brain reorganization involved in the generation of disorders, including the uncertainty for decision-making process. Running Monte Carlo methods typically requires a large number of model evaluations, which demand powerful computer resources and high-performance computing. The higher the number of samples on Monte Carlo methods, the better is the approximation, subsequently, a large demand on computational resources. Facility Hubs such as EBRAIN™ can provide access to supercomputing networks and cloud computing for data simulation, and running Monte Carlo algorithms in parallel. Notably, the application of brain stimulation in Bayesian inference is able to solve the non-identifiability issue to accurately retrieve brain structure-function relationship, and to predict the changes in brain structure as observed in different brain diseases.

The method for diagnosing, assessing the severity or monitoring a brain disorder in a mammal's brain or a condition affecting the mammal's brain, for example epilepsy, Alzheimer, Parkinson, multiple sclerosis, brain tumor including glioblastoma, and a condition resulting from a stroke or a traumatic injury, and that comprises the various steps mentioned above, is intended to be implemented, in particular, using the brain stimulation method and system described above, which implements TI or mTI.

**Claims**

1. A method for inferring a change in a whole-brain network in a mammal's brain, comprising

providing a computerized platform modelling various regions of a mammal's brain and connectivity between these regions;
providing three-dimensional anatomical structural imaging data of the mammal's brain, the data comprising anatomical structural data relating to a structural connectivity in the brain of the mammal;
providing the computerized platform with the anatomical structural imaging data of the mammal's brain;
providing a parametric mathematical neural model describing an evolution of a neural field in a brain of a mammal;
providing magnetic resonance imaging time-series data of the mammal's brain, wherein said magnetic resonance imaging time-series data are acquired under stimulation of at least a specific region of the brain of the mammal;
fitting the model with the magnetic resonance imaging time-series data;
estimating the parameters of the model;
inverting the model; and
inferring the change in the whole-brain network in the mammal's brain.

2. The method of claim 1, wherein the three-dimensional anatomical structural imaging data of the mammal's brain further comprise geometry data extracted from T1-weighted magnetic resonance imaging and wherein the structural connectivity is extracted from tractography on diffusion weighted magnetic resonance imaging data.

3. The method of one of the claims 1 or 2, wherein the magnetic resonance imaging time-series data of the mammal's brain are acquired non-invasively.

4. The method of claim 3, wherein the magnetic resonance imaging time-series data of the mammal's brain are acquired non-invasively by performing a brain stimulation of the specific region of the brain of the mammal.

5. The method of claim 4, wherein, for performing the brain stimulation of the mammal's brain, it is provided at least two pairs of stimulation electrodes (1, 2), each pair of electrodes providing an electric signal at a carrier frequency (f1, f2) generating an envelope frequency for stimulation at a point in space in the brain of the patient (C), the envelope frequency being equal to the difference Δf between the two electric signal frequencies provided.

6. The method of one of the claims 4 or 5, wherein, for performing the stimulation of the mammal's brain, it is provided at least four pairs of stimulation electrodes (1, 2, 3, 4), each pair of electrodes providing an electric signal at a carrier frequency (f1, f2, f3, f4), wherein the mean value between the carrier frequency (f1) of a first pair (1) of electrodes and the carrier frequency (f2) of any second pair (2) of electrodes defines a first mean carrier frequency, and the mean value between the carrier frequency (f3) of a third pair (3) of electrodes and the carrier frequency (f4) of a fourth pair (4) of electrodes defines a second mean carrier frequency, and wherein the difference between said first and second mean carrier frequencies is at least 200 Hz, preferably at least 500 Hz even more preferably at least 800 Hz.

7. The method of one of the claims 5 or 6, wherein each electric signal provided by each pair of electrodes (1, 2, 3, 4) has a carrier frequency (f1, f2, f3, f4) of at least 800 Hz, preferably at least 900 Hz, more preferably at least 1000 Hz.

8. The method of one of the claims 1 to 7, wherein the parameters of the model are estimated by performing an automatic full Bayesian inference.

9. The method of claim 8, wherein the automatic full Bayesian inference is performed using Monte Carlo algorithms.

10. The method of one of the claims 8 or 9, wherein the automatic full Bayesian inference is performed using Probabilistic Programming languages.

11. The method of one of the claims 1 to 10, wherein the model depend on a stimulation (I_stim) to at least the specific brain region.

12. The method of one of the claims 1 to 10, wherein the model is provided by the set of equations as follows:

$$\dot{r}_i(t) \;=\; \frac{\Delta}{\pi} + 2r_i(t)V_i(t)$$

$$\dot{v}_i(t) \;=\; V_i^2(t) - \pi^2 r_i^2(t) + \eta + Jr_i(t) + G\sum_{j=1}^{N} SC_{ij}^*(r_j - r_i)) + I_{stim} + \xi(t),$$

where the variable *r(t)* is the population firing rate of a region *i of the brain,* and *v(t)* is the average membrane potential of the mass in region i, the parameter *J* represents the average synaptic weight, the parameters $\Delta$ and $\eta$ represent the average excitability of the mass neurons and the heterogeneous noise spread, respectively, and the parameter *G* is the global parameter that modulates the overall impact of *SC\** on the state dynamics of each region and represents the disorders in the brain connectome.

13. The method of one of the claims 1 to 12, wherein the parametric neural model describing the evolution of the neural field the brain of the mammal depends on at least a parameter ($\alpha$) that corresponds to the normalized intensity of changes in the structural connectivity and on a global scaling parameter.

14. The method of one of the claims 1 to 13, wherein the three-dimensional anatomical structural imaging data of the mammal's brain are parcelled into discrete regions.

15. The method of claim 14, wherein the regions are non-overlapping.

16. The method according to one of the claims 1 to 15, wherein the change is indicative of a brain disorder or of a condition affecting the mammal's brain, or of a severity of a brain disorder or of a condition affecting the mammal's brain.

17. A method for diagnosing, assessing the severity or monitoring a brain disorder in a mammal's brain or a condition affecting the mammal's brain, comprising

   providing a computerized platform modelling various regions of a mammal's brain and connectivity between these regions;
   providing three-dimensional anatomical structural imaging data of the mammal's brain, the data comprising anatomical structural data relating to a structural connectivity in the brain of the mammal;
   providing the computerized platform with the anatomical structural imaging data of the mammal's brain;
   providing a parametric mathematical neural model describing an evolution of a neural field in a brain of a mammal;
   providing magnetic resonance imaging time-series data of the mammal's brain, wherein said magnetic resonance imaging time-series data are acquired under stimulation of at least a specific region of the brain of the mammal;
   fitting the model with the magnetic resonance imaging time-series data;
   estimating the parameters of the model;
   inverting the model; and
   diagnosing, assessing the severity or monitoring a brain disorder in the mammal's brain or a condition affecting the mammal's brain.

18. The method of one of the claims 16 or 17, wherein the brain disorder or the condition affecting the mammal's brain is a pathologic condition affecting the brain selected from the group consisting in epilepsy, Alzheimer, Parkinson, multiple sclerosis, brain tumor including glioblastoma, and a condition resulting from a stroke or a traumatic injury.

19. A system for inferring a change in a whole-brain network in a brain of a mammal, comprising

   a computerized platform modelling various regions of a mammal's brain and connectivity between these regions;
   three-dimensional anatomical structural imaging data of the brain of the mammal, the data comprising anatomical structural data relating to a structural connectivity in the brain of the mammal;

the computerized platform being provided with the anatomical structural imaging data of the brain of the mammal;

a parametric mathematical neural model describing an evolution of the neural field in a brain of a mammal;

magnetic resonance imaging time-series data of the brain of the mammal, wherein said magnetic resonance imaging time-series data are acquired under stimulation of at least a specific region of the brain of the mammal;

the model being fitted with the magnetic resonance imaging time-series data;

means for estimating the parameters of the model;

means for inverting the model; and

means for inferring the change in the whole-brain network in the mammal's brain.

FIG. 1

**A. TI : Adding two frequencies create an envelope**

**B. mTI : Adding two envelopes create a greater envelope**

**C. mTI : Reducing the overall amplitude increases focality**

Increase in focality

Fig. 2A

Optimal interference area

Fig. 2B

FIG. 2C

Fig. 2D

SC healthy

FIG. 3A

FIG. 3B

mask inter-hem

mask intra-hem

FIG. 3C

SC disordered

FIG. 3D

FIG. 4A

FIG. 4B

FIG. 4C

FIG. 4D

FIG. 4E

Europäisches
Patentamt

European
Patent Office

Office européen
des brevets

# EUROPEAN SEARCH REPORT

**Application Number**

EP 23 16 9081

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/359060 A1 (LEE JIN HYUNG [US] ET AL) 10 November 2022 (2022-11-10) <br> * paragraphs [0043] – [0058] * <br> * paragraphs [0084] – [0088] * <br> * paragraphs [0107] – [0115] * <br> * figures * | 1-19 | INV. <br> A61B5/00 <br><br> ADD. <br> A61N1/04 |
| A | US 2022/039736 A1 (JIRSA VIKTOR [FR] ET AL) 10 February 2022 (2022-02-10) <br> * paragraphs [0007], [0008] * <br> * paragraphs [0019] – [0039] * | 2,8-10, 14,15 | |
| A | SE 2 150 968 A1 (FRIGG AB [SE]) 24 January 2023 (2023-01-24) <br> * paragraphs [0006], [0042] – [0058] * <br> * claims; figures * | 5-7 | |
| A | JIRSA V K ET AL: "The Virtual Epileptic Patient: Individualized whole-brain models of epilepsy spread", NEUROIMAGE, vol. 145, 15 January 2017 (2017-01-15), pages 377-388, XP029856190, ISSN: 1053-8119, DOI: 10.1016/J.NEUROIMAGE.2016.04.049 <br> * the whole document * | 1,19 | TECHNICAL FIELDS SEARCHED (IPC) <br><br> A61B <br> G16H <br> A61N |

The present search report has been drawn up for all claims

| Place of search | Date of completion of the search | Examiner |
|---|---|---|
| The Hague | 20 September 2023 | Bataille, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 9081

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2023

| Patent document cited in search report | | Publication date | Patent family member(s) | | Publication date |
|---|---|---|---|---|---|
| US 2022359060 | A1 | 10-11-2022 | AU | 2020360399 A1 | 21-04-2022 |
| | | | CA | 3156760 A1 | 08-04-2021 |
| | | | CN | 114981818 A | 30-08-2022 |
| | | | EP | 4038550 A1 | 10-08-2022 |
| | | | IL | 291887 A | 01-06-2022 |
| | | | JP | 2022550476 A | 01-12-2022 |
| | | | KR | 20220150874 A | 11-11-2022 |
| | | | US | 2022359060 A1 | 10-11-2022 |
| | | | WO | 2021067464 A1 | 08-04-2021 |
| US 2022039736 | A1 | 10-02-2022 | CA | 3030238 A1 | 25-01-2018 |
| | | | CN | 109640810 A | 16-04-2019 |
| | | | EP | 3484355 A1 | 22-05-2019 |
| | | | ES | 2919141 T3 | 22-07-2022 |
| | | | JP | 7132555 B2 | 07-09-2022 |
| | | | JP | 2019527105 A | 26-09-2019 |
| | | | US | 2019254585 A1 | 22-08-2019 |
| | | | US | 2022039736 A1 | 10-02-2022 |
| | | | WO | 2018015778 A1 | 25-01-2018 |
| SE 2150968 | A1 | 24-01-2023 | SE | 2150968 A1 | 24-01-2023 |
| | | | WO | 2023003501 A1 | 26-01-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROXIN.** Macroscopic description for networks of spiking neurons. *Physical Review X,* 2015, vol. 5 (2), 021028